(19) Europäisches Patentamt
European Patent Office
Office européen des brevets

(11) **EP 4 512 315 A1**

(12) **EUROPEAN PATENT APPLICATION**

(43) Date of publication:
**26.02.2025   Bulletin 2025/09**

(51) International Patent Classification (IPC):
**A61B 5/021** $^{(2006.01)}$ **A61B 5/022** $^{(2006.01)}$
**A61B 5/00** $^{(2006.01)}$

(21) Application number: **23192495.2**

(22) Date of filing: **21.08.2023**

(52) Cooperative Patent Classification (CPC):
**A61B 5/022; A61B 5/02108; A61B 5/7203;
A61B 5/7221; A61B 5/7239; A61B 5/7242;
A61B 5/7246; A61B 5/7278**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **DERKX, Rene Martinus Maria
5656 AG Eindhoven (NL)**
• **WIJSHOFF, Ralph Wilhelm Christianus Gemma
Rosa
5656 AG Eindhoven (NL)**

(74) Representative: **Philips Intellectual Property &
Standards
High Tech Campus 52
5656 AG Eindhoven (NL)**

(54) **ARTEFACT ANALYSIS SYSTEM**

(57)     A method for artefact detection and analysis in a signal, particularly a signal received from a pressure sensor of a hemodynamic measurement apparatus comprising a compressing cuff. It is proposed to acquire a pressure signal as a function of time during a measurement period in which an applied pressure to a body part is varied through a range of applied pressures. Analysis is performed of a morphology change of pulses in the signal as a function of changing applied pressure. A representation of the morphology change of the pulses is extracted through feature analysis applied to the pulses, and this is then fitted to a model, where the model represents an expected morphology change in the absence of artefacts. The residual between the model fit and the actual morphology change is calculated and this is used to determine a severity of artefacts present in the signal.

FIG. 6

FIG. 7

EP 4 512 315 A1

**Description**

FIELD OF THE INVENTION

The present invention relates to a method for signal artefact analysis, in particular within the field of hemodynamic parameter monitoring, for example hemodynamic monitoring using a cuff-based measurement apparatus.

BACKGROUND OF THE INVENTION

Blood pressure and cardiac output (and/or stroke volume) are important hemodynamic parameters in a variety of contexts, including for example in a surgery setting to assess the status of a patient during a surgical procedure.

The most common method for non-invasive blood pressure (NIBP) measurement is the use of an oscillometric blood pressure measurement cuff. The cuff comprises an inflatable bladder. The cuff wraps around the upper arm of a patient and is controlled to progress through an inflation and deflation cycle during which a pressure inside the bladder is sampled and a single set of values for systolic arterial pressure, mean arterial pressure and diastolic arterial pressure are derived through processing of the pressure signal.

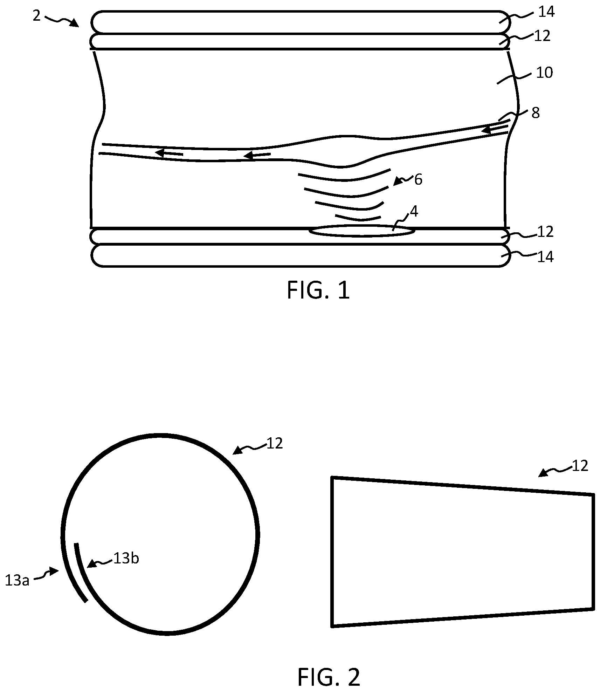

A variant approach to non-invasive blood pressure measurement has previously been proposed. This also uses a cuff which wraps around the body part of the patient. A cross-sectional illustration is shown in Fig. 1. The cuff 2 is shown wrapped around the arm 10 of a patient, and the brachial artery 8. The cuff 2 includes a pressure actuator 14, which optionally may be an inflatable bladder, as in the traditional NIBP cuff. In variance with the traditional NIBP cuff, the variant cuff also includes a separate tissue pressure sensor 4 arranged to measure a pressure between the body part 10 and the cuff. The tissue pressure sensor in some examples comprises a fluid-filled sensor pad, wherein a pressure in the pad varies as a function of pressure exerted on the pad. Also in variance with the traditional NIBP cuff, the variant cuff includes a hard shell 12 which extends around the body part and is disposed (radially) between the actuator 14 and the body part 10.

The pulse waves in the brachial artery 8 cause a change in the tissue movement. This change in tissue movement causes a compression of the liquid in the sensor pad 4. The sensor pad is fluidly connected via a flexible tube filled with similar liquid to a pressure transducer which measures the pressure signal.

The pressure sensor pad 4 is enclosed by a hard shell 12 to enable stiffness and enable a sufficient signal from the pressure sensor. In similarity with a standard NIBP cuff, the pressure actuator 14 may comprise a bladder which is inflatable with an air pump, to compress the arm tissue, so that the brachial artery 8 can be closed.

Due to the inclusion of the separate tissue pressure sensor 4 with hard shell 12 backing, this cuff can obtain a high fidelity pulse signal for the patient, and, from this, multiple different hemodynamic parameters can be obtained, including stroke volume and cardiac output.

The shell structure 12 has an annular form, and, in the most common design, is formed from a single piece of material which is curled round to span a complete (closed) loop. Fig. 2 schematically shows an example shell structure 12 according to the state of the art. Fig. 2 (left) shows a cross-section across a plane normal to a longitudinal axis of the shell and parallel with tangential and radial axes of the shell. Fig. 2 (right) shows a cross-section across a plane parallel with a longitudinal axis of the shell.

The shell structure is for extending around the body part 10 and is for arrangement between the tissue pressure sensor and the pressure actuator during operation, the shell structure being discontinuous circumferentially so as to having at least one pair of tangentially overlapping edge surfaces 13a, 13b which are tangentially slidable relative to one another during operation.

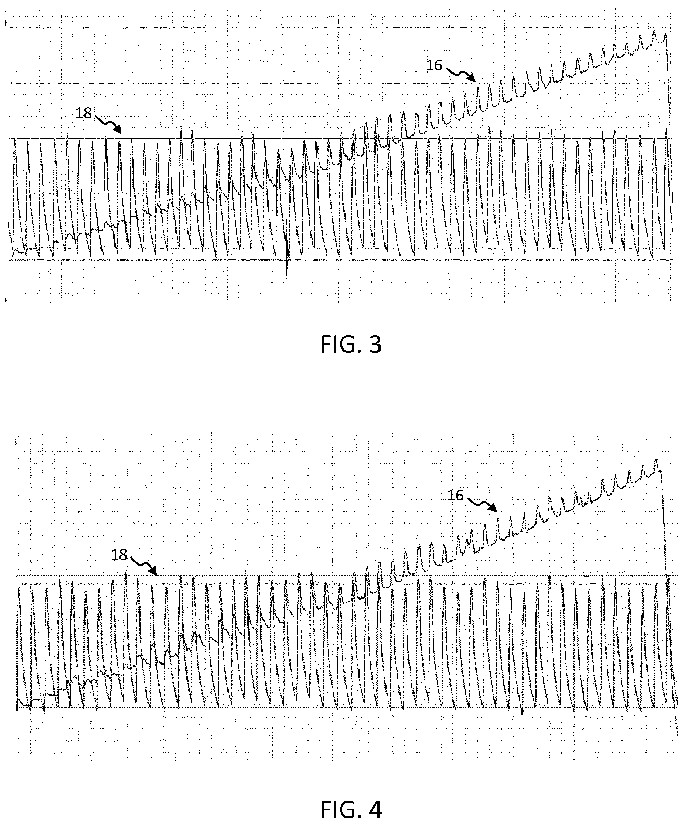

Fig. 3 shows a portion of a real-world tissue pressure signal 16 acquired from the tissue pressure sensor 4 of a shell-backed cuff of the type described above during a measurement cycle in which the pressure applied by the pressure 14 actuator ramps upward through a series of increasing values. For reference, Fig. 3 further shows arterial pressure 18 as measured simultaneously for the same patient over the same time period using an (invasive) arterial line ("A-line"). A-line measurement is the current clinical gold standard for arterial pressure measurement and is measured using a thin catheter inserted into an artery.

It may be observed from Fig. 3 that the heart pulsations in the tissue pressure signal 16 show a changing morphology over time. In particular, while earlier initial pulsations (up to Mean Arterial Pressure (MAP)) show a fairly smooth exponential decay of the diastole part in the pulsations, the pulsations above MAP show a more flattened diastole phase. In this sense, the pulsations seem to 'sharpen' when the pressurization of the cuff is close to or above the systole pressure.

Fig. 4 shows a portion of a further real-world tissue pressure signal 16 acquired for the same patient from the tissue pressure sensor 4 of a shell-backed cuff of the type described above. As in Fig. 3, Fig. 4 further shows arterial pressure 18 as measured simultaneously for the same patient over the same time period using an (invasive) arterial line. In this example, there was touching of the cuff by the clinician. Due to the sensitivity of the tissue pressure sensor 4, this results in additional artefacts in the form of distortions in the morphology of the signal pulses.

A means for detecting artefacts in the tissue pressure signal, and preferably quantifying a severity of the artefacts would be of value.

SUMMARY OF THE INVENTION

It is the recognition of the inventors that, in the absence of artefacts, the pulse waveforms of the tissue pressure signal follow a predictable slow morphology change as a function of changing applied pressure by the cuff. It is the proposal of the inventors to take advantage of this property to detect artefact severity by measuring a deviation of the signal from the expected smooth morphology change of pulse waveforms.

The invention is defined by the claims.

According to examples in accordance with an aspect of the invention, there is provided a method for signal artefact analysis, comprising:

receiving a tissue pressure signal, TP signal, from a tissue pressure sensor arranged to sense a pressure between a pressure actuator of a measurement cuff and tissue of a user wearing the cuff, the TP signal spanning a time period, the time period spanning a plurality of cardiac cycles;

extracting an AC component of the tissue pressure signal to derive an AC tissue pressure signal, TPac;

applying a pre-determined one or more signal conditioning steps, to thereby derive a conditioned signal, CTPac,

analyzing the CTPac to identify a plurality of pulse waveforms and a respective reference point for each identified pulse waveform;

for each respective one of a plurality of time lag time values:

computing a respective set of signal level drop values wherein the set of signal level drop values comprises one signal level drop value for each of the identified pulse waveforms in the CTPac, indicative of a signal level drop between the reference point of the pulse waveform and a point occurring the respective time lag away from the reference point, and optionally further normalized with respect to the amplitude of the CTPac at the reference point, and

determining an n-th order polynomial fit for the respective set of signal level drop values, wherein n is equal to or greater than 1;

to thereby derive a plurality of n-th order polynomial fits, one for each of the lag time values;

forming a model signal, $M^n(t)$, spanning the whole time period, wherein the model signal is formed from values of the plurality of n-th order polynomial fits coinciding with time points spanned by the time period;

computing a residual signal for the TPac signal based on the model signal and the CTPac or the TPac signal; and

determining a measure indicative of artefact severity for the received tissue pressure signal based on the residual signal.

In other words, the model signal is formed by combining the data points of the plurality of n-th order polynomial fits into a single common data series, spanning the time axis. In other words, forming the model signal comprises re-plotting the values from the time lag polynomial model curves as a function of time along a single common time axis. This may be referred within this disclosure as a composite model signal, in the sense that it is formed from a combination of the data points of the plurality of polynomial fits for the different time lag points.

The set of polynomial fits effectively provide a representation of morphology change of the pulse waveforms as a function of time.

This morphology change is known to follow a slow and smooth progression, such that it can be modelled well by a polynomial fit.

The present invention is based on exploiting this characteristic to detect deviation in a real signal from such a smooth, slow morphology change, this being used as a proxy indicator for presence of artefacts.

It is proposed in particular to perform a respective polynomial model fit for each of a set of phase points of the pulses as a function of pulse index, and, from this, construct a model signal for the whole time period by re-plotting the values from those curves on a common axis as a function of time.

This represents a best fit of the received data to an expected polynomial-like progression of pulse waveform morphology as a function of time.

Deviation of the actual tissue pressure signal from this model fit provides a proxy indicator for artefact presence and artefact severity.

The AC tissue pressure signal oscillates around the x=0 axis.

Optionally the method further comprises determining a normalized CTPac by, for each part of the CTPac corresponding to a respective identified pulse waveform, normalizing said part of the CTPac with respect to the amplitude of the CTPac at the identified reference point for said cardiac cycle, and wherein the remainder of the steps of the method are applied to the normalized CTPac signal.

The output of the fitting process is the set of coefficients defining the polynomial.

With regards to the signal conditioning, in some embodiments, the one or more signal conditioning steps include integrating the TPac.

The systolic peaks in the original tissue pressure signal are not always consistently at the right position. For example, due to the mentioned morphology-change at applied pressure close to systolic arterial pressure (SAP), there may temporarily appear two peaks in the TPac signal which makes the construction of the model very difficult.

In some embodiments, the one or more signal conditioning steps further comprise negating the signal after integration, such that the CTPac signal is a negated integral signal of the TPac signal.

Here, it is proposed to integrate the TPac signal together with a negation such that the peaks of the integrated and negated signal are aligned with the end-of-diastole time points. As a result, the different pulse waves in the model are aligned at this end-of-diastole moment.

As a consequence, each 'pulse waveform' referred to previously is in fact a pulse waveform in the negated integral signal, where the peak of each pulse corresponds to an end diastole moment of a heart cycle.

The aforementioned reference point may be a peak point, i.e. a maximum.

Given that the signal conditioning is applied before the model signal formation, in some embodiments the method may further comprise performing second signal conditioning, the second signal conditioning applied to the formed model signal, $M^n(t)$, in advance of computing the residual signal, to thereby yield a conditioned model signal, $CM^n(t)$. The second signal conditioning may for example comprise a set of one or more signal conditioning steps which represent a reverse of the pre-determined one or more signal conditioning steps applied to derive the conditioned tissue pressure signal. In order words, the second signal conditioning may 'undo' or revert or invert the signal conditioning applied to the TPac signal before the steps to derive the model signal were performed.

Where such second signal conditioning is performed, the residual signal may be computed based on computing a difference between the conditioned model signal, CM(t) and the original TPac signal (before the one or more conditioning steps were applied). Where such second signal conditioning is not applied, the residual signal may be computed based on computing a difference between the model signal $M^n(t)$ and the CTPac signal (i.e., after signal conditioning steps were applied).

More explicitly, with regards to the second conditioning, where the one or more signal conditioning steps include integrating the TPac, wherein the second signal conditioning may comprise a step of differentiating the model CTPac signal before computing the residual signal.

By way of further example, where the one or more signal conditioning steps comprise negating the signal after integration, such that the CTPac signal is a negated integral signal of the TPac signal, the second signal conditioning may comprise steps of negating the model signal, $M^n(t)$, followed by differentiating the negated model signal before computing the residual signal.

In some embodiments, the one or more signal conditioning steps applied to yield the CTPac signal may further comprise performing trend compensation on the negated integral signal based on determining a trend compensation signal and subtracting the trend compensation signal from the negated integral signal.

In some embodiments, for each lag time value, the polynomial model fit may be performed as a weighted polynomial regression, wherein a weighting applied to each term of the polynomial regression sum is determined as a function of the strength of the pulse waveform to which that respective term relates. For example, the strength of the pulse can be the peak (systolic) value of the pulse waveform or the peak-to-valley (systolic to diastolic) value of the pulse waveform.

As is well known, weighted polynomial regression is a form of polynomial regression in which different data points are given different weights in the regression calculation.

The weights are included in the least squares minimization procedure that is used to estimate the parameters of the regression model. Regression fitting involves minimizing the sum of a set of squared residuals, where each residual refers to the difference between the observed and predicted values of a certain data series (in this case the TP signal, or a conditioned version thereof). In weighted regression, each squared residual is further multiplied by a respective weight before being summed and minimized. This gives more influence to data points with higher weights and less influence to data points with lower weights.

As discussed above, the method comprises a step of determining a residual signal for the tissue pressure signal based on the model signal and the CTPac signal, or based on the conditioned model signal (where second signal conditioning is applied) and the TPac signal.

For example, the residual signal may be computed by taking a difference between the original CTPac(t) signal and the model signal, $M^n(t)$ (the composite model signal):

$$\text{residual(t)} = \text{CTPac(t)} - \text{M}^n(\text{t})$$

where CTPac(t) is the conditioned AC component of the tissue pressure signal, and $M^n(t)$ is the model tissue pressure signal.

Alternatively, where the second signal processing is applied before computing the residual signal, then the residual signal may be computed by taking a difference between the original TPac(t) signal and the conditioned model signal, $CM^n(t)$:

$$\text{residual(t)} = \text{TPac(t)} - \text{CM}^n(\text{t})$$

In some embodiments, the measure of artefact severity may be determined based on a maximum value of the residual signal.

In some embodiments, the measure of artefact severity may be determined based on an aggregation of the values of the residual signal. The aggregation may for example comprise one or more of: mean, mean absolute error, root mean square error.

For avoidance of doubt, mean absolute error (MAE) is an average of the absolute values of the residual signal. It provides a measure of the magnitude of the errors, irrespective of their direction. Root Mean Square Error (RMSE) is the square root of the average of the squared residual signal values. By squaring the residual signal values before averaging, this metric also ignores the direction of the errors and places more weight on larger errors.

In some embodiments, the determining the measure of a magnitude of artefact severity in the received signal comprises computing a confidence value, conf, using any of the equations:

$$conf = 1 - \frac{\max\left(|\text{CTPac} - \text{M}^n(\text{t})|\right)}{\max\left(\text{CTPac[peaks]}\right)}$$

or

$$conf = 1 - \frac{\text{average}(|\text{CTPac} - \text{M}^n(\text{t})|)}{\max\left(\text{CTPac[peaks]}\right)}$$

or

$$conf = 1 - \frac{\text{rms}(|\text{CTPac} - \text{M}^n(\text{t})|)}{\max\left(\text{CTPac[peaks]}\right)}$$

where CTPac is the conditioned AC component of the tissue pressure signal, $M^n(t)$ is the model tissue pressure signal, and CTPac[peaks] represents the set of peak values of the conditioned AC component of the tissue pressure signal. For avoidance of doubt "rms" refers to root mean square error.

Thus, in the first equation, the numerator represents a maximum value of the absolute value of the residual signal, in the second equation the numerator represents an average value of the absolute value of the residual signal and in the third equation the numerator represents a root mean square error of the (absolute value of the) residual signal.

Alternatively, in cases where the second signal conditioning is applied to the model signal before computing the residual signal, then determining the measure of a magnitude of artefact severity in the received signal may comprise computing a confidence value, conf, using any of the equations:

$$conf = 1 - \frac{\max\left(|\text{TPac} - \text{CM(t)}|\right)}{\max\left(\text{TPac[peaks]}\right)}$$

or

$$conf = 1 - \frac{\text{average}(|\text{TPac} - \text{CM(t)}|)}{\max\left(\text{TPac[peaks]}\right)}$$

or

$$conf = 1 - \frac{\text{rms}(|\text{TPac} - \text{CM(t)}|)}{\max\,(\text{TPac[peaks]})}$$

where TPac is the AC component of the tissue pressure signal, $CM^n(t)$ is the conditioned model signal, and TPac[peaks] represents the set of peak values of the AC component of the tissue pressure signal.

Here again, in the first equation, the numerator represents a maximum value of the absolute value of the residual signal, in the second equation the numerator represents an average value of the absolute value of the residual signal and in the third equation the numerator represents a root mean square error of the (absolute value of the) residual signal.

In some embodiments, the method further comprises windowing the TP signal in advance of conditioning the signal such that analysis is performed on only a sub-section of the received tissue pressure signal. The window may for example be chosen to coincide with a portion of the cuff inflation cycle known to have high sensitivity to artefacts on the measurement result of the hemodynamic parameter. The particular portion of the cycle to window may be pre-determined in advance.

In some embodiments, the step of identifying the plurality of pulse waveforms and a respective reference point for each identified pulse waveform comprises: receiving an indication of locations of peak points in the TPac signal; defining a searching window around the indicated location of each peak point; and detecting the reference point and pulse waveform by searching within said searching window. For example, the system may include a hemodynamic parameter computation module which is adapted to compute one or more hemodynamic parameters, such as SAP, DAP, MAP, and/or cardiac output, based on the TPac signal, and wherein the hemodynamic parameter computation module as part of the algorithm employed thereby detects peaks of the TPac signal. For example, this may be done in order to determine an envelope signal, from which at least a subset of the hemodynamic parameters are computed. Thus, this detection of the peaks may be communicated to the artefact analysis method/module for use in streamlining the detection of the pulses in the CTPac signal.

In some embodiments, the method comprises controlling a user interface to generate user feedback indicative of the magnitude of artefact severity.

In some embodiments, an absolute value of each of the lag time values is equal to or less than a half of a duration of a cardiac cycle of the subject.

In some embodiments, the reference point for each identified pulse waveform is a peak.

Another aspect of the invention is a computer program product comprising computer-executable code configured, when run by a processor, to cause the processor to perform a method in accordance with any embodiment described in this disclosure, or in accordance with any claim of this application.

Another aspect of the invention is a processing device, comprising: an input/output; and one or more processors configured to:

receive (e.g., at the input/output) a tissue pressure signal, TP signal, from a tissue pressure sensor arranged to sense a pressure between a pressure actuator of a measurement cuff and tissue of a user wearing the cuff, the TP signal spanning a time period, the time period spanning a plurality of cardiac cycles;
extract an AC component of the tissue pressure signal to derive an AC tissue pressure signal, TPac;
apply a pre-determined one or more signal conditioning steps, to thereby derive a conditioned TPac signal, CTPac, analyze the CTPac signal to identify a plurality of pulse waveforms and a respective reference point for each identified pulse waveform;
for each respective one of a plurality of time lag time values:

compute a respective set of signal level drop values wherein the set of signal level drop values comprises one signal level drop value for each of the identified pulse waveforms in the CTPac, indicative of a signal level drop between the reference point of the pulse waveform and a point occurring the respective time lag away from the reference point, and optionally further normalized with respect to the amplitude of the CTPac at the reference point, and
determine an n-th order polynomial fit for the respective set of signal level drop values, wherein n is equal to or greater than 1,
to thereby derive a plurality of n-th order polynomial fits, one for each of the lag time values;

form a model signal spanning the time period, wherein the model signal is formed from values of the plurality of n-th order polynomial fits coinciding with the time points spanned by the time period;
compute a residual signal based on the model signal and the CTPac signal or the TPac signal;

determine a measure indicative of artefact severity for the received tissue pressure signal based on the residual signal.

In other words, the data points of the model signal are populated by the amalgamated data points of the plurality of n-th order polynomial fits replotted onto a common time axis.

Another aspect of the invention is a system, comprising: a processing device in accordance with any embodiment described in this document or in accordance with any claim of this application; and

a blood pressure measurement apparatus comprising: a cuff for wrapping around a body part of a user, and wherein the cuff includes a pressure actuator for use in applying a varying pressure to the body part by the cuff; and a tissue pressure sensor arranged for sensing a pressure between a surface of the body part and the cuff.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example hemodynamic parameter measurement apparatus suitable for use as part of embodiments of the present invention;

Fig. 2 shows schematic representations of a shell portion of the apparatus of Fig. 1;

Fig. 3 shows an example tissue pressure signal illustrating the morphology change of pulse waves of the signal as a function of applied pressure;

Fig. 4 shows an example tissue pressure signal having artefacts in the signal;

Fig. 5 outlines steps of an example method in accordance with one or more embodiments of the invention;

Fig. 6 is a block diagram of an example system in accordance with one or more embodiments of the invention;

Fig. 7 is a block diagram further illustrating a processing flow in accordance with one or more embodiments;

Fig. 8 shows an example tissue pressure signal AC component, TPac;

Fig. 9 shows an example set of pulse waveforms of the TPac signal aligned with one another in time;

Fig. 10 shows a plot of signal level drop values for one lag time point as a function pulse wave index, j;

Fig. 11 illustrates pulse waveforms from an example composite model signal for a low-artefact situation; and

Fig. 12 illustrates pulse waveforms from an example composite model signal for a high-artefact situation.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for artefact detection and analysis in a signal, particularly a signal received from a pressure sensor of a hemodynamic measurement apparatus comprising a compressing cuff. It is proposed to acquire a pressure signal as a function of time during a measurement period in which an applied pressure to a body part is varied through a range of applied pressures. Analysis is performed of a morphology change of pulses in the signal as a function of changing applied pressure. A representation of the morphology change of the pulses is extracted through feature analysis applied to the pulses, and this is then fitted to a model, where the model represents an expected morphology change in the absence of artefacts. The residual between the model fit and the actual morphology change is calculated and this is used to determine a severity of artefacts present in the signal.

Extracting a representation of morphology change of the pulses is a challenge. The inventors propose to use a simple methodology which may be summarized as follows. At each of a series of defined sample point locations along the phase of each pulse in the signal: (a) compute a signal level drop metric indicative of a signal level drop between the sample point and a reference point of the pulse; (b) fit the signal level drop values for the series of pulses to a pre-defined model fit curve, e.g. a polynomial, (c) to thereby derive a respective model curve for each of said defined sample point locations representing variation of the signal level drop at that sample point location as a function of pulse index, and (d) from these plurality of model curves, it is proposed to construct a composite model signal for the whole time window by re-plotting the values from the model curves for the plurality of sample point locations as a function of time along a single common time axis. This process will become clearer with explanations later.

At least one motivation behind the making of the present invention is to provide a means for assessing artefact presence within the tissue pressure signal, or a processed or conditioned version thereof, output from a measurement cuff of the type discussed earlier in this document with reference to Fig. 1 and Fig. 2. The details and structure of this cuff will not be repeated again, for brevity.

Since the morphology of the targeted heart pulsations in the signal change slowly over time, the inventors propose a model that takes advantage of this slowly varying morphology. By subtracting the fitted model wave from the original wave, there may be determined a measure indicative of an amount of energy in the signal that is caused by external artefacts (i.e., non physiological, and not related to the heart pulsations).

As mentioned, the proposed solution exploits the slowly changing morphology in the heart pulsations of the tissue-pressure waveform (measured by the pressure-pad 4 of the cuff - see Fig. 1) visible throughout the inflation.

By subtracting the composite model signal from the original tissue-pressure waveform (or a conditioned version thereof), a residual signal is obtained which ideally contains all of the artefacts caused by non-physiological sources, e.g., external disturbances.

In some embodiments, if the external disturbances exceed a pre-defined threshold, this may trigger a response action, such as outputting advice to a user flagging the measurement as containing artefacts and thus flagging the algorithm output parameters as unreliable/inaccurate.

It is noted that the proposed method for assessing artefact presence and severity can be combined with any other types of artefact assessment method.

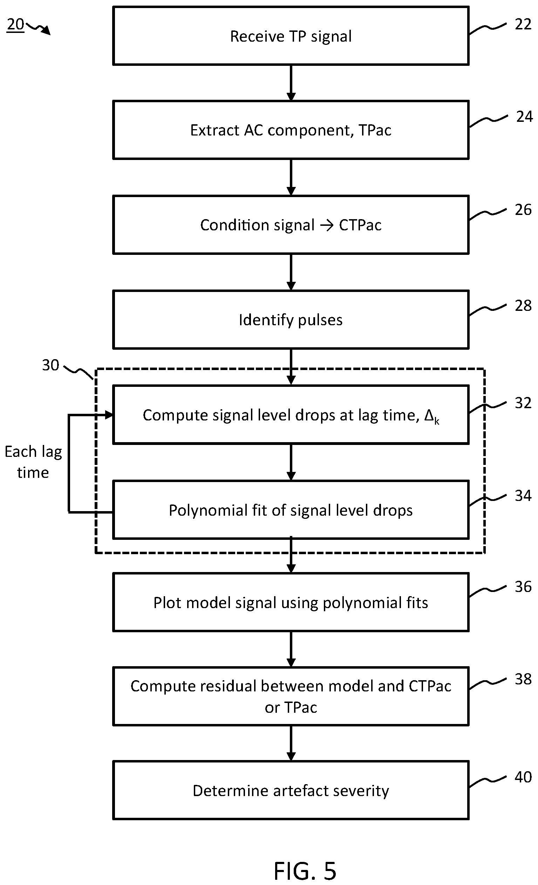

Fig. 5 outlines in block diagram form steps of an example method according to one or more embodiments. The steps will be recited in summary, before being explained further in the form of example embodiments.

The method 20 comprises receiving 22 a tissue pressure signal, TP signal, from a tissue pressure sensor arranged to sense a pressure between a pressure actuator of a measurement cuff and tissue of a user wearing the cuff, the TP signal spanning a time period, the time period spanning a plurality of cardiac cycles.

The method further comprises extracting 24 an AC component of the tissue pressure signal to derive an AC tissue pressure signal, TPac. The resulting TPac comprises just the (higher frequency) oscillation part of the original TP signal, with the baseline trend (indicative of the changing cuff applied pressure) removed. As a result, the TPac signal comprises a series of pulses or pulse waves which each represent an oscillation about the x=0 axis.

The method further comprises applying 26 a pre-determined one or more signal conditioning steps, to thereby derive a conditioned TPac signal, CTPac.

By way of example, the signal conditioning steps may comprise computing an integral of the TPac signal. The signal conditioning steps may further comprise negating the integral of the TPac signal to yield a negated integral signal.

Additionally or alternatively, the signal conditioning steps may include normalization of the pulses comprised by the TPac signal.

The method further comprises analyzing the CTPac to identify 28 a plurality of pulse waveforms and a respective reference point for each identified pulse waveform. The reference point may in some embodiments be a peak of the respective pulse waveform.

The method further comprises, for each respective one of a plurality of time lag time values, performing the following operation 30: (a) computing 32 a respective set of signal level drop values wherein the set of signal level drop values comprises one signal level drop value for each of the identified pulse waveforms in the CTPac, indicative of a signal level drop between the reference point of the pulse waveform and a point occurring the respective time lag away from the reference point, and optionally further normalized with respect to the amplitude of the CTPac at the reference point, and (b) determining 34 an n-th order polynomial fit for the respective set of signal level drop values, wherein n is equal to or greater than 1. This operation 30 is performed for each of a plurality of time lag values. This results in a plurality of n-th order polynomial fits, one for each of the time lag values.

The method further comprises forming 36 a model tissue pressure signal, $M^n(t)$, spanning the whole time period, wherein the model signal is formed from values of the plurality of n-th order polynomial fits coinciding with time points spanned by the time period. In other words, the model signal is formed by combining the data points of the plurality of n-th order polynomial fits into a single common data series, spanning the time axis. The model signal might otherwise be referred to as a composite model signal, because it is formed from compositing the data points of the set of polynomial fits into a single data series.

The method further comprises computing 38 a residual signal for the tissue pressure signal based on the model signal, $M^n(t)$, and either the CTPac signal or the TPac signal.

The method further comprises determining 40 a measure indicative of artefact severity for the received tissue pressure signal based on the residual signal.

As noted above, the method can also be embodied in hardware form, for example in the form of a processing device which is configured to carry out a method in accordance with any example or embodiment described in this document, or in accordance with any claim of this application.

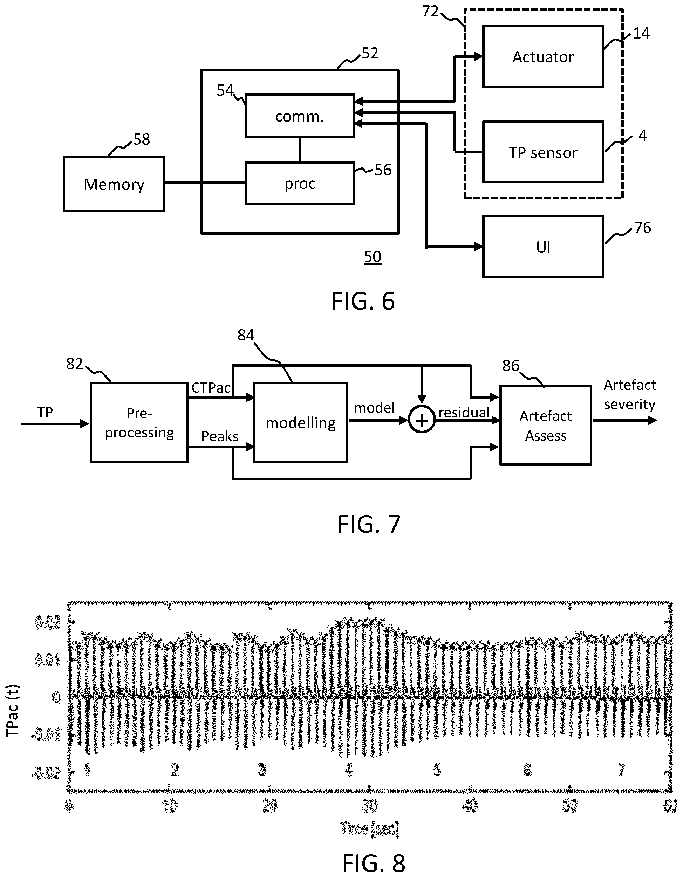

To further aid understanding, Fig. 6 presents a schematic representation of an example processing device 52 configured to execute a method in accordance with one or more embodiments of the invention. The processing device is shown in the context of a system 50 which comprises the processing device. The processing device alone represents an aspect of the invention. The system 50 is another aspect of the invention. The provided system need not comprise all of the illustrated hardware components; it may comprise only a subset of them.

The processing device 52 comprises one or more processors 56 configured to perform a method in accordance with that outlined above, or in accordance with any embodiment described in this document or any claim of this application. In the illustrated example, the processing device further comprises an input/output 54 or communication interface.

In the illustrated example of Fig. 6, the system 50 further comprises a blood pressure measurement apparatus 72 comprising a cuff for wrapping around a body part of a user, and wherein the cuff includes a pressure actuator 14 for use in applying a varying pressure to the body part by the cuff. The apparatus further incorporates a tissue pressure sensor 4 arranged for sensing a pressure between a surface of the body part and the cuff.

The system 50 may further comprise a user interface 76 for use in generating user feedback indicative of the magnitude of artefact severity.

The system 50 may further comprise a memory 58 for storing computer program code (i.e. computer-executable code) which is configured for causing the one or more processors 56 of the processing unit 52 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

A diagram illustrating the processing flow of the method is presented in Fig. 7. The diagram shows pre-processing 82 applied to the tissue pressure, TP, signal, including the extraction of the AC component, TPac, and the signal conditioning, to derive conditioned signal CTPac. Extraction of the AC component removes the linear cuff inflation ramp. Also illustrated is an optional step of processing the TP signal to detect the (systolic) pulse peaks in the signal. These can be used as part of the subsequent model fitting operation to aid in detecting the reference points, e.g. peaks, of the conditioned signal CTPac. Modelling 84 is performed using the conditioned signal, CTPac comprising performing the polynomial fits discussed earlier for each of the plurality of lag times. This will be discussed in greater detail later. The output of the modelling 84 procedure is a composite model signal, $M^n(t)$. A residual between this and the original TPac signal or CTPac signal is then computed, for example based on subtraction. Artefact assessment 86 is then performed based on the residual.

With regards to the signal conditioning, as mentioned previously, in some embodiments, the one or more signal conditioning steps include integrating the TPac, to yield an integral signal. In some embodiments, the one or more signal conditioning steps further comprise negating the signal after integration, such that the CTPac signal is a negated integral signal of the TPac signal.

In some embodiments, in view of the signal conditioning applied before the model signal formation, second signal conditioning may be performed after the model signal formation, which may be configured to be the inverse of the first signal conditioning steps.

In other words, in some embodiments, second signal conditioning may be applied to the formed model signal, $M^n(t)$, in advance of computing the residual signal, to thereby yield a conditioned model signal, $CM^n(t)$. The second signal conditioning may for example comprise a set of one or more signal conditioning steps which represent a reverse of the pre-determined one or more signal conditioning steps applied to derive the conditioned tissue pressure signal. In order words, the second signal conditioning may 'undo' or revert or invert the signal conditioning applied to the TPac signal before the steps to derive the model signal were performed.

Where such second signal conditioning is performed, the residual signal may be computed based on computing a difference between the conditioned model signal, CM(t) and the original TPac signal (before the one or more conditioning steps were applied). Where such second signal conditioning is not applied, the residual signal may be computed based on computing a difference between the model signal $M^n(t)$ and the CTPac signal (i.e. after signal conditioning steps were applied).

More explicitly, with regards to the second conditioning, where the one or more signal conditioning steps include integrating the TPac, the second signal conditioning may comprise a step of differentiating the model CTPac signal before computing the residual signal.

By way of further example, where the one or more signal conditioning steps comprise negating the signal after integration, such that the CTPac signal is a negated integral signal of the TPac signal, the second signal conditioning may comprise steps of negating the model signal, $M^n(t)$, followed by differentiating the negated model signal before computing the residual signal.

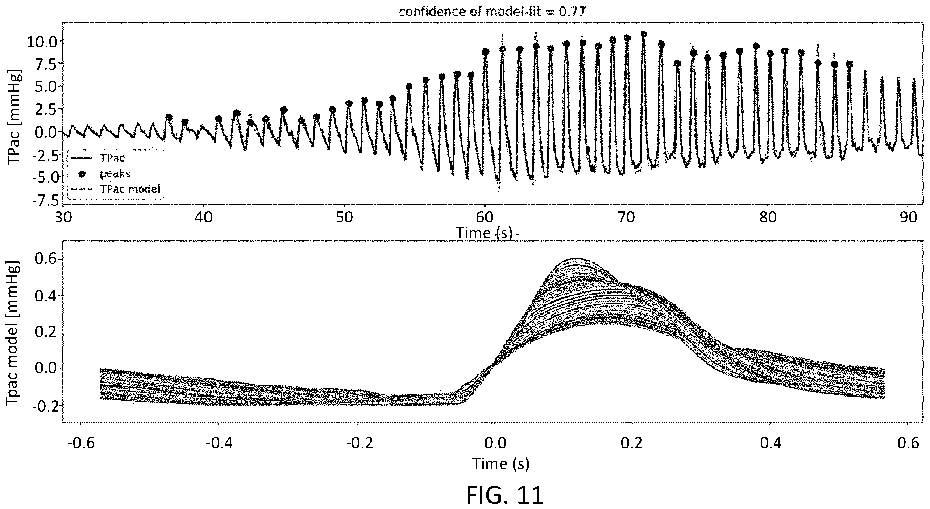

The reason for taking the integral of the TPac as part of the signal conditioning (and the optional differentiation post-processing at the end) is because the systolic peaks detected from the TP signal are not always (consistently) at the correct position. Because of the mentioned morphology-change at the inflation pressure close to SAP, there can occur (temporarily in the inflation) two peaks in the TPac signal, which makes the construction of the model difficult. Hence, the TPac signal may be integrated (together with a negation) such that the peaks of the integrated (and negated signal) are aligned with the end-of-diastole moment. As a result, the different waves in the model are aligned at this end-of-diastole moment, as can be observed in the lower pane of Fig. 11.

The one or more signal conditioning steps may further comprise performing trend compensation on the negated integral signal based on determining a trend compensation signal and subtracting the trend compensation signal from the negated integral signal. The trend compensation signal may be computed for example by a sliding moving-average computation with a (2N+1)-order window that is computed symmetrically [-N,..N], where the order is chosen such that (2N+1)/Fs is set to multiple seconds (i.e. spanning multiple pulse cycles). A further possibility to derive a trend compensation signal would be to perform a first-order polynomial fit over the entire time duration of the CTPac signal.

It is noted that if the signal conditioning steps applied to derive the CTPac signal include such trend compensation, then the optional second signal conditioning applied to the model signal, $M^n(t)$ may also include a reverse trend compensation applied to the model signal.

With regards to the step of computing the measure of artefact severity, there are a variety of options.

In some embodiments, it may be computed based on a maximum value of the residual signal.

Additionally or alternatively, the measure of artefact severity may be determined based on an aggregation of the values of the residual signal.

By way of example, the aggregation may comprise one of: the mean, the mean absolute error, or the root mean square error.

By way of a more specific example, the determining the measure of a magnitude of artefact severity in the received signal may comprise computing a confidence value, *conf*, using any of the equations:

$$conf = 1 - \frac{\max\left(|CTPac - M^n(t)|\right)}{\max\left(CTPac[peaks]\right)}$$

or

$$conf = 1 - \frac{\text{average}(|CTPac - M^n(t)|)}{\max\left(CTPac[peaks]\right)}$$

or

$$conf = 1 - \frac{\text{rms}(|CTPac - M^n(t)|)}{\max\left(CTPac[peaks]\right)}$$

where CTPac is the conditioned AC component of the tissue pressure signal, $M^n(t)$ is the model tissue pressure signal, and CTPac[peaks] represents the set of peak values of the conditioned AC component of the tissue pressure signal. For avoidance of doubt "rms" refers to root mean square error.

Alternatively, in cases where the second signal conditioning is applied to the model signal before computing the residual signal, then determining the measure of a magnitude of artefact severity in the received signal may comprise computing a confidence value, conf, using any of the equations:

$$conf = 1 - \frac{\max\left(|TPac - CM(t)|\right)}{\max\left(TPac[peaks]\right)}$$

or

$$conf = 1 - \frac{\text{average}(|TPac - CM(t)|)}{\max\left(TPac[peaks]\right)}$$

or

$$conf = 1 - \frac{\text{rms}(|\text{TPac} - \text{CM(t)}|)}{\max(\text{TPac[peaks]})}$$

where TPac is the AC component of the tissue pressure signal, $CM^n(t)$ is the conditioned model signal, and TPac[peaks] represents the set of peak values of the AC component of the tissue pressure signal.

In some embodiments, the method may comprise initially windowing the TPac signal in advance of conditioning the signal such that analysis is performed on only a sub-section of the received tissue pressure signal. The window may for example be chosen to coincide with a portion of the cuff inflation cycle known to have high sensitivity to artefacts.

For example, in the context of an inflatable cuff, some derived hemodynamic parameters may be most sensitive to artefacts in the earlier part of the inflation cycle and some of the parameters may be most sensitive to artefacts in the later part of the inflation cycle.

Different artefact severity assessments might be performed for the different hemodynamic parameters in some embodiments.

As discussed above, the method comprises a step of deriving from the CTPac signal a model signal based on plotting a plurality of polynomial fits to lag time points of the plurality of pulses in the CTPac signal. This procedure will now be discussed in more detail.

By way of introduction, as mentioned previously, the method is based on using an expected morphology change of the pulse waveforms in the measured signal, or a conditioned version thereof, as a function of increasing applied pressure to detect artefacts via computing a deviation of the measured signal from that expected morphology change.

To explain further, an example will be considered of a TP signal spanning a time window of 1 minute and which includes 74 pulse waveforms, each corresponding to a cardiac cycle.

As discussed above, the TP signal is processed to extract the AC component, TPac. Fig. 8 illustrates the TPac signal for this example.

For purposes of illustration, the peak of each pulse waveform is indicated by a respective cross in Fig. 8. In some embodiments, the method comprises detecting the peak of each pulse waveform in the TPac signal. This may optionally be used in performing peak detection in the conditioned signal CTPac, e.g. by defining more narrowly a searching window, or defining a start point for a search for each peak.

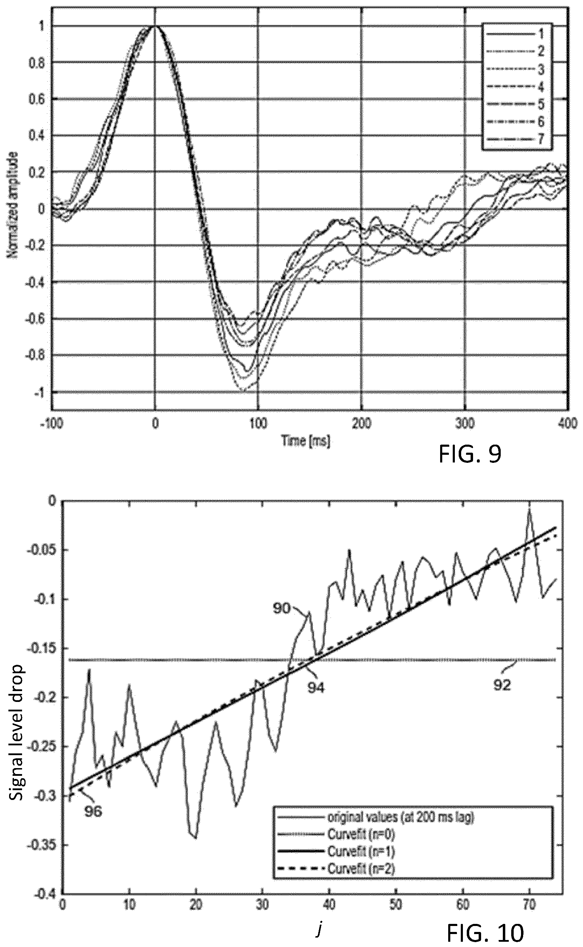

To show how the morphology of the TP signal changes over a measurement cycle (e.g. of 60-second duration), Fig. 9 is a graph illustrating pulse waveforms extracted from the TPac signal, and overlaid with each other.

The seven pulse waveforms included in Fig. 9 are labelled 1 to 7 in Fig. 9, and they are relatively evenly spaced through the 1-minute time window. The time points of these seven waveforms are also indicated with numbers 1-7 in the plot of Fig. 8. It should be noted that these 7 cardiac cycles have been arbitrarily chosen from the cardiac cycles identified in Fig. 8 simply to provide a representation of how the pulse waveform morphology can change. The full method may in fact utilize all of the pulse waveforms spanning the time window.

In Fig. 9, the pulse waveforms for the 7 selected cardiac cycles are aligned in time such that a common reference point of each waveform (in this case, the peak) occurs at t=0. The pulse waveforms have all been normalized so that the amplitude of each pulse waveform at the reference point are the same (amplitude = 1), and at other times the pulse waveforms have respective amplitudes that are usually in the range of -1 to 1.

The time measured from the aligned reference points is referred to as the "lag time" or "lag time value", and is denoted $\Delta_k$, with the aligned reference points corresponding to lag time $\Delta_k$ of 0 seconds. Each of the 7 pulse waveforms shown in Fig. 9 may be referred to as 'normalized' pulse waveforms.

It can be seen from Fig. 9 how the complete morphology of the waveforms (after the peak) changes over time.

As discussed previously, the TPac signal may be conditioned with one or more conditioning steps before applying the remainder of the analysis. This results in a conditioned TPac signal, CTPac(t). The conditioning steps may in some embodiments include taking an integral of the TPac signal and optionally also negating the integrated signal.

For describing the algorithm hereafter, a vector $\underline{x}$ is defined as the CTPac signal data in a time window with size of $N_x$ samples, corresponding to a duration of $N_x/F_s$ seconds, where $F_s$ is the sampling rate of the TP signal. The reference points (which in the following worked example are the peak locations) within the waveform $\underline{x}$ are listed as a vector, $\underline{p}$, that has a length $N_p$, where $N_p$ is the number of cardiac cycles or number of identified reference points. In the presently described example, $N_p$ is 74. The waveform values at the detected peak locations are denoted by

$$\underline{x}_{(\underline{p})_j} \triangleq \left(\underline{x}_p\right)_j$$

where $j = 0, \ldots, N_p-1$ is the index of the pulse waveform.

For detecting the reference point (peak in this case) locations in the CTPac signal, optionally this may be performed using information about detected peak locations in the original TPac signal. For example, the step of identifying the plurality of pulse waveforms and a respective reference point for each identified pulse waveform in CTPac may comprise: receiving indication of locations of peak points in the TPac signal; defining a searching window around the indicated location of each peak point; and detecting the reference point and pulse waveform by searching within said searching window. This simplifies the process of detecting the peaks in the CTPac signal. Detection of peaks in the TPac signal may for example be a step which is already performed as part of hemodynamic parameter determination.

A next step in the modelling process is to analyze the $N_p$ neighboring samples towards the left and right with respect to the reference points (the peaks in this case) and compute for each neighboring sample the change (signal level drop) compared to the initial peak level.

Similar to the vector $\underline{p}$ used to define the peak locations, the neighboring locations with respect to the peak locations can be defined by a vector,

$$\underline{L} = \underline{p} + \Delta_k$$

where $\Delta_k$ is the lag time with respect to the peak locations, which can either be positive or negative valued, positive values corresponding to neighboring samples to the right of the peak, and negative values corresponding to neighboring samples to the left of the peak. The CTPac signal value at each of the lag time locations may be denoted:

$$\underline{x}_{[(\underline{p})_j + \Delta k]} \triangleq \left(\underline{x}_{p+\Delta k}\right)_j$$

where $\Delta_k$ is the lag time with respect to the peak locations, and $j = 0, \dots , N_p-1$ is the index of the pulse waveform.

For each of the time lag values, $\Delta_k$, in each of the pulse waveforms $j$, a respective signal level drop value, $\Delta x$, is computed, indicative of a signal level drop between the peak (the reference point) of the pulse waveform and a point occurring the respective time lag away from the peak (the reference point). This may be denoted as:

$$\underline{\Delta x}_{[(\underline{p})_j + \Delta k]} = \left(\underline{x}_p\right)_j - \left(\underline{x}\right)_{[(p)_j + \Delta k]}$$

Each signal level drop value may optionally be further normalized with respect to the amplitude of the CTPac at the peak (the reference point), i.e.

$$\underline{\Delta x}_{[(\underline{p})_j + \Delta k]} = \frac{\left(\underline{x}_p\right)_j - \left(\underline{x}\right)_{[(p)_j + \Delta k]}}{\left(\underline{x}_p\right)_j}$$

It will be recognized that this process yields, for each respective one of the plurality of time lag values, $\Delta_k$, a respective set of signal level drop values, $\underline{\Delta x}$, wherein the set of signal level drop values comprises one signal level drop value for each of the identified pulse waveforms (index j) in the CTPac signal, $\underline{x}$.

The method then comprises determining, for each time lag value, $\Delta_k$, an n-th order polynomial fit, $M^n$, for the respective set of signal level drop values spanning over the pulse waveforms, j, wherein n is equal to or greater than 1:

$$M^n(j, \Delta_k) = a_0 + a_1 \cdot \left[(\underline{p})_j + \Delta k\right] + \cdots + a_n \cdot \left[(\underline{p})_j + \Delta k\right]^n$$

In other words, for each polynomial fit, $\Delta_k$ is held constant, and the fit is performed over variable, $j$, i.e. fitting a change or variation in the signal level drop value at a certain time lag away from the peak as a function of incrementing pulse waveform index. This therefore represents how the height difference from the peak at the set time lag point changes across successive pulse waveforms across the whole CTPac signal. Therefore, the independent variable in each polynomial fit is the pulse waveform index, j, and the coefficients are adjusted to find a best fit polynomial for the plot of $\underline{\Delta x}_{[(\underline{p})_j + \Delta k]}$ as a function of j for the respective lag time point, $\Delta_k$.

Performing the polynomial fit may be done using a regression method, for example a least squares regression method, the optimization equation for which may be represented for example by

$$\arg \min_{a_0, \cdots, n} \sum_{j=0}^{N_p-1} \left(\frac{\left(\underline{x}_p\right)_j - \left(\underline{x}\right)_{[(p)_j + \Delta k]}}{\left(\underline{x}_p\right)_j} - M^n(j, \Delta_k)\right)^2$$

Alternatively, a weighted regression method may be performed, wherein for each lag time value, $\Delta_k$, the polynomial model fit is performed as a weighted polynomial regression, wherein a weighting applied to each squared residual term in the optimization equation is determined as a function of peak value of the pulse waveform, j, to which that respective term relates.

As is well known, weighted polynomial regression is a form of polynomial regression in which different data points are given different weights in the regression calculation.

The weights are included in the least squares minimization procedure that is used to estimate the parameters of the regression model. Regression fitting involves minimizing the sum of a set of squared residuals, where each residual refers to the difference between the observed and predicted values of a certain data series. In weighted regression, each squared residual is further multiplied by a respective weight before being summed and minimized. This weight in the present case is determined as a function of (e.g. proportional to) the pulse strength of the pulse waveform in the original tissue pressure signal to which the squared residual term relates. However, there are different options with regard to determining the values for the weights. One approach is to use the systolic peak values in the original TP signal. Another is to use the peak-to-valley amplitude of the pulse in TPac or CTPac. Another would be to use the peak-to-valley amplitude of the normalized pulse in TPac or CTPac.

In other words, the optimization equation may be replaced by:

$$\arg \min_{a_0, \cdots, a_n} \sum_{j=0}^{N_p - 1} w_j \left( \frac{(\underline{x}_p)_j - (\underline{x})_{[(p)_j + \Delta k]}}{(\underline{x}_p)_j} - M^n(j, \Delta_k) \right)^2$$

where $w_j$ are weights, and optionally wherein each weight $w_j$ is determined as a function of (e.g. proportional to) the pulse strength of the pulse waveform j to which the term in the sum relates.

Once all of the polynomial fits have been performed, a final (composite) model CTPac signal, $M^n(t)$, can be formed by simply plotting all of the signal level drop values, $\Delta x$, of $M^n(j, \Delta_k)$ across all j and all $\Delta_k$ onto a common time axis.

As has been explained, a respective polynomial fit is performed for each of a plurality of positive and negative time lag values, $\Delta_k$. Since the model fit only needs to span a systole phase of the heart-cycle, bounds for the negative and positive values of $\Delta_k$ can be applied. For the negative values of $\Delta_k$, optionally only negative values that are part of the start of the systole phase may be included. Since the peak location is very close to this start of the systole, the negative values of $\Delta_k$ might for example be limited to be equivalent to e.g. -0. 1 seconds. For the positive values of $\Delta_k$, lag times that are part of the remainder of the systole might be included. Typical maximum positive values for $\Delta_k$ might for example be chosen to be equivalent to lag times of e.g. 0.4 seconds.

In general, the range of the lag time values may be chosen such that an absolute value of each of the lag time values is equal to or less than a half of a duration of a cardiac cycle of the subject. The range of the lag time values may be chosen such that an absolute value of each of the lag time values is equal to or less than a half of a duration of the shortest (minimum duration) cardiac cycle within the TPac signal.

To illustrate the plurality of polynomial fits, Fig. 10, shows the signal level drop values, $\Delta x$, at one of the plurality of lag times, namely $\Delta_k$ = +200 ms (the lag times being lags with respect to the pulse waveform peaks in this case) for the 74 cardiac cycles of the above example. Fig. 10 shows the signal level drop values both for the original CTPac signal, and for example polynomial fits computed for the respective lag time.

The signal level drop values for the original CTPac signal are indicated by line 90. Thus, each value in line 90 corresponds to the signal level drop value (y-axis) of the CTPac signal at lag time $\Delta_k$ = +200 ms in each of the cardiac cycles within the time window. The x-axis represents pulse waveform index, j. For example, the value of line 90 for the 10th pulse waveform (the 10th cardiac cycle) is the value of the CTPac signal of the 10th cardiac cycle at +200 ms lag time from the 10th pulse waveform peak, the value of line 90 for the 25th cardiac cycle is the value of the CTPac signal of the 25th cardiac cycle at +200 ms lag time from the 25th peak, and so on.

Fig. 10 also shows a 0-th order model CTPac signal, $M^0(j, \Delta_k)$, at lag time $\Delta_k$ = +200 ms (line 92), which represents simply the mean of all signal level drop values at that lag time across the 74 pulse waveforms. Fig. 10 also shows a 1st order model CTPac signal, $M^1(j, \Delta_k)$, at lag time $\Delta_k$ = +200 ms (line 94), and a 2nd order model CTPac signal, $M^2(j, \Delta_k)$, at lag time $\Delta_k$ = +200 ms (line 96).

It can be seen how for the model fits of order 1 and above, the signal level drop values smoothly increment as a function of pulse waveform index, j, this representing a smooth morphology change of the pulse waveforms as a function of increasing applied pressure by the cuff.

It will be recognized that a respective model fit is computed for a range of lag time values, $\Delta_k$. Subsequently, the data points of these plurality of model fits are combined into a single common data series spanning the time window covered by the original CTPac signal to yield a composite model signal, $M^n(t)$.

Once the composite model signal, $M^n(t)$, has been derived, a measure indicative of artefact severity is determined.

Optionally, before deriving the measure indicative of artefact severity, a second set of conditioning steps may be performed. By way of example, steps may be performed which are the inverse or reverse of at least some of the first set of conditioning steps performed on TPac to derive CTPac. For example, if the conditioning steps applied to TPac included integrating the TPac signal, then the composite model signal may be differentiated. If the conditioning steps applied to the TPac signal included integration followed by negation, then the composite model may be negated and differentiated. This process yields a conditioned composite model signal, $CM^n(t)$.

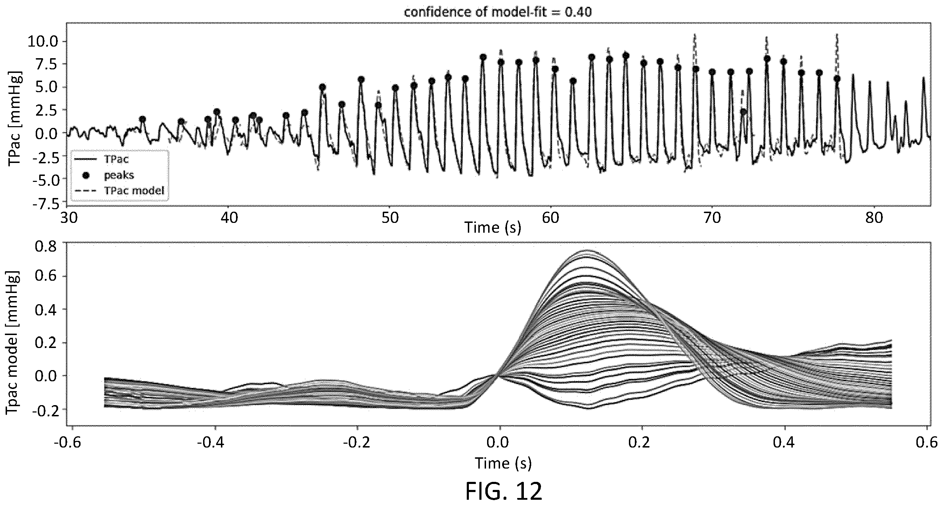

By way of illustration of the process described above, Fig. 11 and Fig. 12 illustrate example (conditioned) model signals computed from TP signals in two different scenarios. Fig. 11 shows a scenario in which there was minimal artefact presence (low artefact energy), while Fig. 12 shows a scenario in which there was significant artefact presence (higher artefact energy). The top plot of each figure shows the original TPac(t) signal overlaid with the final conditioned composite model signal $CM^n(t)$. The original TPac(t) signal is shown with a solid line in each case, while the conditioned model signal $CM^n(t)$ is shown with a dashed line in each case. The bottom plot of each figure shows the individual pulse waveforms of the conditioned model signal $CM^n(t)$ extracted and aligned with one another in time such that the change in morphology as a function of time over the whole signal time window can be seen.

In each case, it can be seen that there are broader pulses visible from the earlier part of the inflation cycle and sharper pulses visible from the later part of the inflation cycle. Thus, the morphology of the pulses changes as applied pressure increases.

For the higher artefact case (Fig. 12), it can be seen that the model fit deviates from the true TPac signal in many places.

In each case, a value for the measure of artefact severity was computed using the previously discussed equation:

$$conf = 1 - \frac{\max\left(|\text{TPac} - \text{CM}^n(\text{t})|\right)}{\max\left(\text{TPac[peaks]}\right)}$$

For the low-artefact case (Fig. 11), the value of *conf* was computed as 0.77. For the higher-artefact case (Fig. 12), the value of *conf* was computed as 0.4.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method (20) for signal artefact analysis, comprising:

    receiving (22) a tissue pressure signal, TP signal, from a tissue pressure sensor arranged to sense a pressure between a pressure actuator of a measurement cuff and tissue of a user wearing the cuff, the TP signal spanning a time period, the time period spanning a plurality of cardiac cycles;
    extracting (24) an AC component of the tissue pressure signal to derive an AC tissue pressure signal, TPac;
    applying (26) a pre-determined one or more signal conditioning steps, to thereby derive a conditioned TPac signal, CTPac,
    analyzing the CTPac to identify (28) a plurality of pulse waveforms and a respective reference point for each identified pulse waveform;
    for each respective one of a plurality of time lag time values:

        computing (32) a respective set of signal level drop values wherein the set of signal level drop values comprises one signal level drop value for each of the identified pulse waveforms in the CTPac, indicative of a signal level drop between the reference point of the pulse waveform and a point occurring the respective time lag away from the reference point, and optionally further normalized with respect to the amplitude of the CTPac at the reference point, and
        determining (34) an n-th order polynomial fit for the respective set of signal level drop values, wherein n is equal to or greater than 1;
        to thereby derive a plurality of n-th order polynomial fits, one for each of the lag time values;

    forming (36) a model signal, $M^n(t)$, spanning the whole time period, wherein the model signal is formed from values of the plurality of n-th order polynomial fits coinciding with time points spanned by the time period;
    computing (38) a residual signal based on the model signal and the CTPac signal or the TPac signal;
    determining (40) a measure indicative of artefact severity for the received tissue pressure signal based on the residual signal.

2. The method of claim 1, wherein the one or more signal conditioning steps include integrating the TPac.

3. The method of claim 2, wherein the one or more signal conditioning steps further comprise negating the signal after integration, such that the CTPac signal is a negated integral signal of the TPac signal.

4. The method of any preceding claim, wherein the method further comprises performing second signal conditioning, the second signal conditioning applied to the model CTPac signal, $M^n(t)$, in advance of computing the residual signal, to thereby yield a conditioned model signal, $CM^n(t)$, wherein the second signal conditioning comprises a set of one or more signal conditioning steps which represent a reverse of the pre-determined one or more signal conditioning steps applied to derive the CTPac signal.

5. The method of claim 4,

    wherein the one or more signal conditioning steps include integrating the TPac signal; and
    wherein the second signal conditioning comprises a step of differentiating the model signal, $M^n(t)$, before computing the residual signal.

6. The method of claim 5,

    wherein the one or more signal conditioning steps further comprise negating the signal after integration, such that the CTPac signal is a negated integral signal of the TPac signal; and
    wherein the second signal conditioning comprises steps of negating the model signal, $M^n(t)$, followed by differentiating the negated model signal before computing the residual signal.

7. The method of any preceding claim, wherein, for each lag time value, the polynomial model fit is performed as a weighted polynomial regression, wherein a weighting applied for each respective signal level drop value of each set of signal level drops values is determined as a function of peak value of the pulse waveform to which that respective value belongs.

8. The method of any preceding claim, wherein the measure of artefact severity is determined based on a maximum value of the residual signal.

9. The method of any preceding claim, wherein the measure of artefact severity is determined based on an aggregation of the values of the residual signal, optionally wherein the aggregation comprises one of: mean, mean absolute error, root mean square error.

10. The method of any of claims 1-9, wherein determining the measure of a magnitude of artefact severity in the received signal comprises computing a confidence value, conf, using any of the equations:

$$conf = 1 - \frac{\max\left(|\text{CTPac} - \text{M}^n(\text{t})|\right)}{\max\left(\text{CTPac[peaks]}\right)}$$

or

$$conf = 1 - \frac{\text{average}(|\text{CTPac} - \text{M}^n(\text{t})|)}{\max\left(\text{CTPac[peaks]}\right)}$$

or

$$conf = 1 - \frac{\text{rms}(|\text{CTPac} - \text{M}^n(\text{t})|)}{\max\left(\text{CTPac[peaks]}\right)}$$

where CTPac is the conditioned AC component of the tissue pressure signal, $\text{M}^n$(t) is the model tissue pressure signal, and CTPac[peaks] represents the set of peak values of the conditioned AC component of the tissue pressure signal.

11. The method of any of claims 4-6, or 7-9 when dependent on any of claims 4-6, wherein determining the measure of a magnitude of artefact severity in the received signal comprises computing a confidence value, conf, using any of the equations:

$$conf = 1 - \frac{\max\left(|\text{TPac} - \text{CM(t)}|\right)}{\max\left(\text{TPac[peaks]}\right)}$$

or

$$conf = 1 - \frac{\text{average}(|\text{TPac} - \text{CM(t)}|)}{\max\left(\text{TPac[peaks]}\right)}$$

or

$$conf = 1 - \frac{\text{rms}(|\text{TPac} - \text{CM(t)}|)}{\max\left(\text{TPac[peaks]}\right)}$$

where TPac is the AC component of the tissue pressure signal, $\text{CM}^n$(t) is the conditioned model signal, and TPac [peaks] represents the set of peak values of the AC component of the tissue pressure signal.

12. The method of any of claims 1-11, further comprising controlling a user interface to generate user feedback indicative

of the magnitude of artefact severity.

13. A computer program product comprising computer-executable code configured, when run by a processor, to cause the processor to perform a method in accordance with any of claims 1-12.

14. A processing device, comprising:

an input/output; and
one or more processors configured to perform a method in accordance with any of claims 1-12:

15. A system, comprising:

a processing device as claimed in claim 14; and
a blood pressure measurement apparatus comprising:

a cuff for wrapping around a body part of a user, and wherein the cuff includes a pressure actuator for use in applying a varying pressure to the body part by the cuff; and
a tissue pressure sensor arranged for sensing a pressure between a surface of the body part and the cuff.

FIG. 1

FIG. 2

FIG. 3

FIG. 4

20 ↘

| Receive TP signal | 22 |

↓

| Extract AC component, TPac | 24 |

↓

| Condition signal → CTPac | 26 |

↓

| Identify pulses | 28 |

↓

30

Each lag time

| Compute signal level drops at lag time, $\Delta_k$ | 32 |

↓

| Polynomial fit of signal level drops | 34 |

↓

| Plot model signal using polynomial fits | 36 |

↓

| Compute residual between model and CTPac or TPac | 38 |

↓

| Determine artefact severity | 40 |

FIG. 5

FIG. 6

FIG. 7

FIG. 8

FIG. 9

FIG. 10

FIG. 11

confidence of model-fit = 0.40

FIG. 12

Europäisches
Patentamt

European
Patent Office

Office européen
des brevets

# EUROPEAN SEARCH REPORT

Application Number

EP 23 19 2495

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 889 133 A (NELSON CRAIG H [US] ET AL) 26 December 1989 (1989-12-26) <br> * abstract * <br> * column 2, line 53 – column 3, line 4 * <br> * column 5, line 57 – column 6, line 45 * <br> * column 7, line 12 – line 29 * <br> * column 8, line 28 – column 9, line 56 * <br> * column 10, line 53 – line 62 * <br> * column 22, line 19 – line 23 * <br> * column 24, line 41 – line 50 * <br> * column 27, line 40 – column 28, line 20 * <br> * figures 5-9 * | 1,2,8, 13-15 | INV. A61B5/021 A61B5/022 A61B5/00 |
| A | US 2014/073959 A1 (RODRIGUEZ-LLORENTE FERNANDO [GB] ET AL) 13 March 2014 (2014-03-13) <br> * abstract * <br> * paragraphs [0425] – [0428] * | 1-15 | |
| A | WO 2021/260082 A1 (REDWAVE MEDICAL GMBH [DE]) 30 December 2021 (2021-12-30) <br> * abstract * <br> * page 33 – page 36 * <br> * page 42; figure 26 * | 1-15 | **TECHNICAL FIELDS SEARCHED (IPC)** <br><br> A61B |
| A | US 2014/073957 A1 (RODRIGUEZ-LLORENTE FERNANDO [GB] ET AL) 13 March 2014 (2014-03-13) <br> * abstract * <br> * paragraph [0390] * | 1-15 | |
| A | US 2016/228069 A1 (DERKX RENE MARTINUS MARIA [NL] ET AL) 11 August 2016 (2016-08-11) <br> * abstract * <br> * paragraphs [0036], [0071] * | 1-15 | |

The present search report has been drawn up for all claims

| Place of search | Date of completion of the search | Examiner |
|---|---|---|
| The Hague | 7 February 2024 | Delval, Christophe |

EPO FORM 1503 03.82 (P04C01)

**EP 4 512 315 A1**

## ANNEX TO THE EUROPEAN SEARCH REPORT
## ON EUROPEAN PATENT APPLICATION NO.

**EP 23 19 2495**

This annex lists the patent family members relating to the patent documents cited in the above-mentioned European search report.
The members are as contained in the European Patent Office EDP file on
The European Patent Office is in no way liable for these particulars which are merely given for the purpose of information.

**07-02-2024**

| Patent document cited in search report | | Publication date | Patent family member(s) | | Publication date |
|---|---|---|---|---|---|
| US 4889133 | A | 26-12-1989 | NONE | | |
| US 2014073959 | A1 | 13-03-2014 | NONE | | |
| WO 2021260082 | A1 | 30-12-2021 | DE 102021116373 | A1 | 30-12-2021 |
| | | | EP 4171369 | A1 | 03-05-2023 |
| | | | US 2023263411 | A1 | 24-08-2023 |
| | | | WO 2021260082 | A1 | 30-12-2021 |
| US 2014073957 | A1 | 13-03-2014 | NONE | | |
| US 2016228069 | A1 | 11-08-2016 | CN 105578960 | A | 11-05-2016 |
| | | | EP 3048972 | A1 | 03-08-2016 |
| | | | JP 6557219 | B2 | 07-08-2019 |
| | | | JP 2016537037 | A | 01-12-2016 |
| | | | US 2016228069 | A1 | 11-08-2016 |
| | | | WO 2015044010 | A1 | 02-04-2015 |

EPO FORM P0459

For more details about this annex : see Official Journal of the European Patent Office, No. 12/82